# EUROPÄISCHE PATENTANMELDUNG

(11) **EP 0 796 637 A2**
(43) Veröffentlichungstag der Anmeldung: **24.09.1997**
(21) Anmeldenummer: 97103480.6
(22) Anmeldetag: 04.03.1997
(51) Int. Cl.: A61N 5/06, A61G 12/00, A61F 7/00

(54) **Verfahren zur Erwärmung einer Liegefläche für Personen und Bestrahlungsvorrichtung**

(30) Priorität: 22.03.1996 DE 19611251
(71) Anmelder: Heraeus Med GmbH, D-63450 Hanau (DE)
(72) Erfinder: Hartge, Jörg, Dr., 63571 Gelnhausen (DE); Scheller, Walter, 63477 Maintal (DE); Steckhan, Markus, 61440 Oberursel (DE); Greif, Stefan, 36039 Fulda (DE); Czernin, Georg, 63739 Aschaffenburg (DE)
(74) Vertreter: Kühn, Hans-Christian

(57) **Zusammenfassung**

Zur Erwärmung von Liegeflächen für Personen ist eine Bestrahlungsvorrichtung vorgesehen, die Halogen-Glühlampen aufweist, welche mit ihrer Strahlungsrichtung zur Liegenoberfläche zwecks Erwärmung gerichtet sind; die im Bestrahlungsbereich der Liegenfläche herrschende Oberflächentemperatur wird als langwellige Infrarot-Strahlung von einem pyrometrischen Sensor als Regelgröße aufgenommen und mit einem vorgegebenen Sollwert - beispielsweise 37°C - verglichen und bei Regelabweichung wird mittels Stellsignal die Intensität der Bestrahlung durch Ansteuerung der Stromversorgung für die Halogen-Lampen nachgestellt.

Die zugehörige Bestrahlungseinrichtung dient zur Vorerwärmung von Patientenbetten, in die Patienten nach einer schweren Operation wieder eingebracht werden.

## Beschreibung

Die Erfindung betrifft ein Verfahren zur Erwärmung einer Liegefläche für Personen, insbesondere für Patienten im Krankenhaus, wobei die Liegefläche durch wenigstens eine im räumlichen Abstand angeordnete Strahlenquelle bestrahlt wird, sowie eine Bestrahlungsvorrichtung.

Aus der gattungsbildenden EP 254 838 B1 ist eine Bestrahlungseinrichtung zur permanenten Wärmeversorgung einer der Aufnahme von Personen dienenden Liegefläche bekannt; hierzu sind kleinflächige Wärmestrahler vorgesehen, die im vertikalen räumlichen Abstand über den Eckpunkten eines den Liegebereich umfassenden Vielecks angeordnet sind.

Als problematisch erweist es sich, daß zur Markierung des Strahlungsbereiches zusätzliche Lichtstrahler eingesetzt werden müssen, um das eigentliche Bestrahlungsfeld kenntlich zu machen; darüber hinaus ist die Wärmeaufnahme erheblichen äußeren Störungen unterworfen, wie beispielsweise Spannungsschwankungen in der Stromversorgung der Strahler, veränderliche Umgebungstemperaturen und Unregelmäßigkeiten im Wärmeaustausch zwischen der bestrahlten Fläche und der Umgebung.

Darüber hinaus ist eine solche Bestrahlungseinrichtung zur Vorerwärmung von Liegeflächen in Form von Patientenbetten kaum geeignet, da die bekannte Bestrahlungseinrichtung für eine dauerhatte Patienten- bzw. Säuglingsbestrahlung vorgesehen ist und ein einfacher Wechsel von zu bestrahlenden Liegeflächen gegenüber der Bestrahlungseinrichtung nicht vorgesehen ist.

Weiterhin ist aus der EP 275 817 B1 ein elektrisches Lichtbad zur flächenhaften Wärmebestrahlung eines menschlichen Körpers bekannt, welches wenigstens eine Infrarotquelle enthält; durch das Strahlerelement wird vorwiegend eine Strahlung im Wellenlängenbereich zwischen 716 und 1300 nm erzeugt, wobei stabförmige Infrarot-Strahlerelemente in einem mit Reflektoren versehenen Gehäuse angeordnet sind, die ggf. mittels Glashülle bzw. Filteranordnungen sichtbares Licht reduzieren; bei Anwendung ist es möglich, die Bestrahlungsintensität mittels Regelgerät individuell einzustellen.

Aufgrund von Lamellen und Zusatzreflektoren im Strahlungsaustrittsbereich ist eine solche Vorrichtung für die Erwärmung von Liegeflächen in Krankenhausbetten nur unzureichend geeignet, da einerseits zusätzliche Lamellen und Reflektoren vom Pflegepersonal als bedienungsunfreundlich empfunden würden, andererseits auch eine relativ große Ausdehnung der Strahler mit geringer Bündelfähigkeit der Strahlung in Kauf zu nehmen wäre.

Aufgabe der Erfindung ist es Liegeflächen für Personen vorzuwärmen, ohne eine direkte oder indirekte Bestrahlung der Person vorzunehmen; insbesondere sollen auch vorbenutzte Krankenhausbetten, in die Patienten nach schwerer Operation wieder eingebracht werden, vorgewärmt werden, um eine Kreislaufbeeinträchtigung durch Auskühlung in einem üblichen Bett zu verhindern.

Die Aufgabe wird verfahrensgemäß durch die kennzeichnenden Merkmale des Anspruchs 1 gelöst.

Bevorzugte Ausgestaltungen des Verfahrens sind in den Ansprüchen 2 und 3 angegeben.

Als besonders vorteilhaft erweist sich die Regelung der Strahlungsintensität mittels Sensor, der die von der Liegefläche ausgehende Strahlung erfaßt und als Temperaturmeßwert dem Eingang eines Reglers zuführt, wobei die Vorwärmung hohen hygienischen Anforderungen entspricht. Weiterhin ist es vorteilhafterweise möglich, äußere Störeinflüsse zu minimieren.

Die Aufgabe wird vorrichtungsgemäß durch die kennzeichnenden Merkmale des Anspruchs 4 gelöst.

In einer bevorzugten Ausgestaltung der Bestrahlungsvorrichtung ist als Temperaturstrahler eine Halogenglühlampe eingesetzt, deren Hauptstrahlungsanteil einen Spektralbereich unterhalb 2500 nm aufweist.

Weitere vorteilhafte Ausgestaltungen der Bestrahlungsvorrichtung sind in den Ansprüchen 6 bis 8 angegeben.

Ein wesentlicher Vorteil ist in der Möglichkeit des Verschließens der Abstrahlöffnung mittels glattflächiger Abschlußscheibe zu sehen; es sind relativ große Abstände von über 1 m zum Bett möglich, woraus sich eine feste Montierbarkeit an der Halterung ohne Behinderung des Personals in Kopfhöhe - beispielsweise an einer Seitenwandhalterung - ergibt.

Weiterhin ist aufgrund der pyrometrischen Messung ein Ausgleich von Temperatur- oder auch Spannungs-Schwankungen bzw. verschiedener Abstände zwischen Bett und Strahleranordnung möglich.

Aufgrund seines Strahlungsanteils im sichtbaren Spektrum ist die bestrahlte Fläche auf dem Bett für Pflegekräfte eindeutig erkennbar.

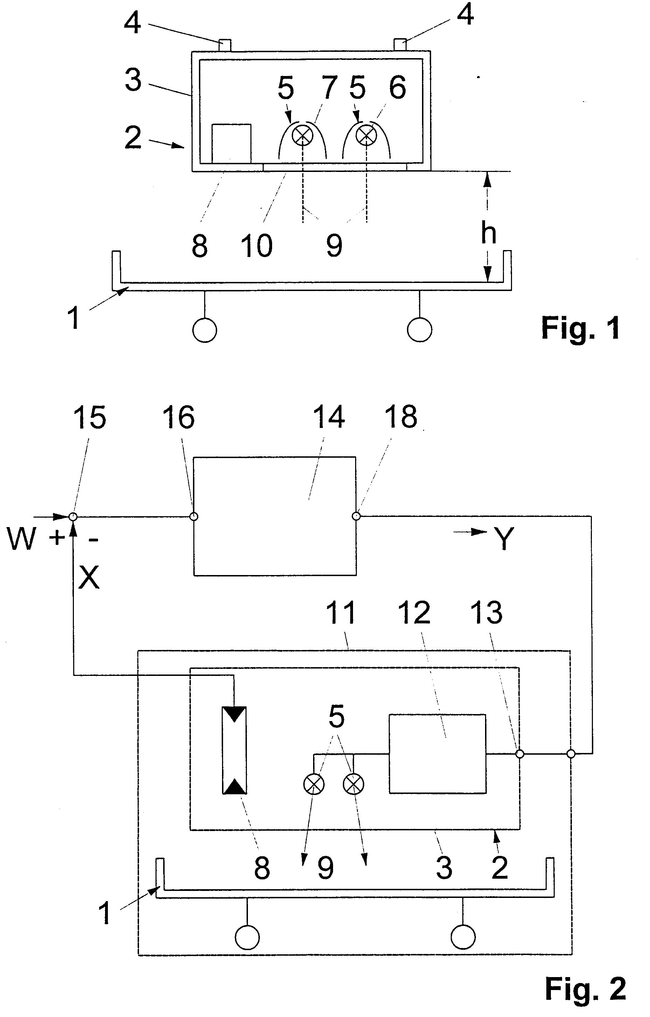

Im folgenden ist der Gegenstand der Erfindung anhand der Figuren 1 und 2 näher erläutert.
Figur 1 zeigt in einer schematischen Anordnung einen Längsschnitt durch die Bestrahlungsanordnung mit der Zugehörigen Liegefläche;
Figur 2 zeigt einen zugehörigen Regelkreis zur Einstellung der Strahlungsintensität in schematischer Darstellung.

Gemäß Figur 1 ist Liege 1 im Strahlungsbereich einer Bestrahlungsvorrichtung 2 angeordnet, wobei Bestrahlungsvorrichtung 2 und Liege 1 gegeneinander beweglich ist. Im vorliegenden Ausführungsbeispiel ist die Bestrahlungsvorrichtung 2 ortsfest angeordnet, während die Liege 1 verschiebbar ist; vorteilhafterweise ist das Gehäuse 3 der Bestrahlungsvorrichtung 2 oberhalb der Kopfhöhe des Pflegepersonals an einer senkrechten Wand mittels Halterung 4 montiert. Die Bestrahlungsvorrichtung 2 enthält neben den schematisch dargestellten Strahlern 5 mit Halogen-Lampen 6 und Reflektor 7 einen pyrometrischen Sensor 8, der ebenfalls schematisch dargestellt ist. Der pyrometrische Sensor 8, befindet sich ebenfalls im Gehäuse 3 der Bestrahlungsvorrichtung 2 und ist zur Aufnahme der von der Liegenoberfläche aufgrund ihrer Temperatur ausgehenden langwelligen IR-Strahlung (Wellenlängenbereich ca. 10 µm) vorgesehen; er weist beispielsweise eine Thermosäule als Meßelement auf. Es ist jedoch auch möglich, Bolometer oder andere Wärmestrahlungs-Detektoren, wie sie beispielsweise in der DE 39 26 859 A1 beschrieben sind, einzusetzen. Die Strahler 5 sind mit ihrer Strahlerachse 9 zur Liege 1 hin ausgerichtet, wobei die Strahlenachsen 9 lediglich symbolisch dargestellt sind.

An der zur Liege 1 gerichteten Unterseite des Bestrahlungs-Gehäuses 3 ist eine Strahlenaustrittsscheibe 10 vorgesehen, welche die Strahlenaustrittsöffnungen der Strahler 5 bzw. die zur Liege 1 gerichtete Strahlenaustrittsseite des Gehäuses 3 gegenüber der Umgebung abschließt. Sensor 8 ist ebenfalls an der Unterseite des Bestrahlungs-Gehäuses 3 angeordnet und ebenfalls zur Oberfläche der Liege 1 ausgerichtet.

Der Abstand zwischen der Unterseite des Bestrahlungs-Gehäuses 3 und der Liege 1 ist variabel und wird mit "h" bezeichnet.

Die Stromversorgung der Bestrahlungseinrichtung erfolgt über ein stationäres Versorgungsnetz, wobei gegebenenfalls eine zusätzliche Not-Stromversorgung vorgesehen ist.

Gemäß Figur 2 ist die Bestrahlungseinrichtung 2 als Teil einer Regelstrecke 11 ausgebildet, zu der Bestrahlungs-Gehäuse 3, Liege 1, Sensor 8 und das zur Stromversorgung der Strahler 5 vorgesehene Stellglied 12 gehören. Die Strahler 5 bzw. die Halogen-Lampen 6 sind mit dem Stellglied 12 als Stromversorgungsgerät verbunden, welches von Regler 14 ein Stellsignal y über Eingang 13 erhält, und somit die Strahlungsintensität auf Liege 1 vorgibt; zur Messung der Oberflächentemperatur der Liege 1 dient der Sensor 8, welcher die in Form von langwelliger Strahlung (Wärmestrahlung) ermittelte Temperatur als Regelgröße x dem Vergleichspunkt 15 am Eingang 16 des Reglers 14 zuführt; gleichzeitig liegt am Vergleichspunkt 15 das Sollwertsignal w für die Temperaturvorgabe - beispielsweise ein Signal für 37°C für die Körpertemperatur - auf der Liegefläche. Die sich aus Differenz von Regelgröße x und Sollwert w ergebende Regelabweichung (w-x) wird dem Eingang 16 des Reglers 14 zugeführt, wobei sie zu einem Stellsignal y gewandelt wird, welches am Ausgang 18 des Reglers 14 abgegeben wird. Stellsignal y wird dann dem der Stromversorgung der Strahler 5 dienenden Stellglied 12 über Eingang 13 zugeführt.

Als besonders vorteilhaft erweist es sich, daß mittels des Regelkreises auf Stellglied 12 einwirkende Störungen des Versorgungsnetzes, der Umgebungstemperatur, Luftströmung und Abstand zwischen Bestrahlungsgerät und Liegefläche sich ergebende Variationen als Störgrößen in den Regelkreis eingehen und bei Regelabweichung mittels Stellsignal ausgeglichen werden können.

Weiterhin erweist es sich als vorteilhaft, daß die Bestrahlungsvorrichtung aufgrund der optisch erkennbaren Bestrahlungsfläche dem Pflegepersonal einen eindeutigen Hinweis auf das vorgewärmte Gebiet der Liegenoberfläche gibt.

## Patentansprüche

1. Verfahren zur Erwärmung einer Liegefläche für Personen, insbesondere für Patienten im Krankenhaus, wobei die Liegefläche durch wenigstens eine im räumlichen Abstand angeordnete Strahlenquelle bestrahlt wird, dadurch gekennzeichnet, daß die Liegefläche von wenigstens einem Temperaturstrahler, dessen Hauptstrahlungsanteil im Spektralbereich bei einer Wellenlänge unterhalb von 2500 nm liegt, bestrahlt wird, wobei zur Einstellung der Intensität der Bestrahlung ein von der Oberflächentemperatur der Liegefläche abhängiges Signal langwelliger IR-Strahlung als Regelgröße aufgenommen und mit einem vorgegebenen Sollwert verglichen wird und bei Regelabweichung mittels Stellsignal die Intensität der Bestrahlung nach dem Prinzip eines geschlossenen Regelkreises nachgestellt wird.

2. Verfahren nach Anspruch 1, dadurch gekennzeichnet, daß die Liegefläche von einer Halogenglühlampe bestrahlt wird.

3. Verfahren nach Anspruch 1 oder 2, dadurch gekennzeichnet, daß zur Ermittlung der Oberflächentemperatur von der Liegeoberfläche ausgehende langwellige IR-Strahlung im Wellenlängenbereich von 6 bis 16 µm von einem Sensor aufgenommen wird.

4. Bestrahlungsvorrichtung zur Erwärmung einer Liegefläche für Personen, insbesondere für Patienten im Krankenhaus, wobei über der Liegefläche im räumlichen Abstand wenigstens ein Strahler zur Wärmeerzeugung angeordnet ist, dadurch gekennzeichnet, daß als Strahler (5) wenigstens ein Temperaturstrahler mit schwarzer Temperatur im Bereich bis 3400 K vorgesehen ist, wobei der Strahler zur Einstellung der abgegebenen Strahlungsintensität mit einem steuerbaren Stromversorgungsgerät verbunden ist, dessen Eingang zur Aufnahme von Stell-Signalen mit dem Ausgang (18) eines Reglers (14) verbunden ist, wobei der Regler an seinem Eingang (16) mit einem Sensor (8) zur Ermittlung einer von der Liegefläche abgegebenen Wärmestrahlung als Regelgröße verbunden ist.

5. Bestrahlungsvorrichtung nach Anspruch 4, dadurch gekennzeichnet, daß als Temperaturstrahler eine Halogenglühlampe (6) eingesetzt wird, die einen Hauptstrahlungsanteil in einem Spektralbereich unterhalb 2500 nm aufweist.

6. Bestrahlungsvorrichtung nach Anspruch 5, dadurch gekennzeichnet, daß die Halogenglühlampe (6) in einem Reflektor (7) angeordnet ist, wobei im Bereich des Strahlenaustritts eine Lampe und Reflektor gegenüber der Liegefläche abschließende Strahlenaustrittsscheibe (10) aus thermisch beständigem Glas oder aus Glaskeramik vorgesehen ist.

7. Bestrahlungsvorrichtung nach Anspruch 4 oder 5, dadurch gekennzeichnet, daß als Sensor (8) zur Aufnahme langwelliger IR-Strahlung ein Pyrometer vorgesehen ist.

8. Bestrahlungsvorrichtung nach Anspruch 7, dadurch gekennzeichnet, daß als Pyrometer eine Thermosäule oder ein Bolometer eingesetzt wird.
